# EUROPÄISCHE PATENTSCHRIFT

(11) **EP 0 400 288 B1**
(45) Veröffentlichungstag und Bekanntmachung des Hinweises auf die Patenterteilung: **25.10.1995**
(21) Anmeldenummer: 90105285.2
(22) Anmeldetag: 21.03.1990
(51) Int. Cl.: A61B 17/39

(54) **Bipolares Koagulationsinstrument**
Bipolar coagulation apparatus
Appareil de coagulation bipolaire

(30) Priorität: 27.05.1989 DE 3917328
(43) Veröffentlichungstag der Anmeldung: 05.12.1990
(73) Patentinhaber: Richard Wolf GmbH, 75438 Knittlingen (DE)
(72) Erfinder: Melzer, Andreas, D-6200 Wiesbaden (DE); Nahrun, Markus, D-5253 Lindlar (DE); Kipfmüller, Karl, Dr., D-6204 Taunusstein (DE); Reidenbach, H.-D., Prof. Dr., D-5206 Neunkirchen-Seelscheid 1 (DE); Buess, Gerd, Prof. Dr., D-6501 Nieder-Olm (DE)
(74) Vertreter: Wilcken, Thomas, Dipl.-Ing.

(56) Entgegenhaltungen:
- CH-A- 547 103
- DE-A- 2 930 982
- US-A- 2 002 594
- US-A- 2 011 169

## Beschreibung

Die Erfindung geht von einem bipolaren Koagulationsinstrument nach der CH-A-547 103 aus.

Dieses Dokument offenbart ein bipolares Koagulationsinstrument mit einem Außenschaft, der distal mit zwei im Abstand zueinander angeordneten Maulteilhälften eines ersten Maulteiles und mit zwei im Abstand zueinander angeordneten Maulteilhälften eines zweiten Maulteiles versehen ist und gegenüber dem die Maulteile axial verschiebbar sind, mit einem ein distal offenes Ende aufweisenden Spülkanal und mit einem Innenschaft, der einen weiteren Kanal aufweist, durch den eine Schneidzange oder dergleichen zwischen den beiden Maulteilen hindurchführbar ist.

Dieses bekannte Koagulationsinstrument kann unter anderem durch einen Instrumentenkanal eines Mediastinoskopes zum Koagulieren von Blutgefäßen und Lymphabflußbahnen hindurchgeführt werden, worauf nach dem Koagulieren das Koagulationsinstrument entfernt und durch eine Schneidzange ersetzt werden muß, um den koagulierten Gewebeteil zu durchtrennen. Es ist also vor allem beim Freipräparieren der Speiseröhre von umgebendem Gewebe ein häufiger Austausch eines Koagulationsinstrumentes gegen eine Schneidzange und umgekehrt erforderlich, was für den Arzt eine erhebliche Belastung bedeutet und für den Patienten zu Beschwerden führt.

Die Aufgabe der vorliegenden Erfindung besteht darin, ein Koagulationsinstrument so zu gestalten, daß die Koagulation von Blutgefäßen und Lymphabflußbahnen an zwei in einem Abstand voneinander liegenden Stellen durchgeführt werden kann und die Gefäße zwischen den beiden Koagulationsstellen durchtrennt werden können, wobei ein häufiger und damit den Arzt und den Patienten belastender Austausch des Koagulationsinstrumentes gegen eine Schneidzange und umgekehrt vermieden werden soll.

Diese Aufgabe wird durch die Merkmale des Anspruches 1 gelöst.

Es ist durch diese Lösung möglich, einen Instrumentenkanal vorhandener Endoskope, vor allem eines Mediastinoskopes, für die Hindurchführung der bipolaren Koagulationszange durch einen Instrumentenkanal zu verwenden und die Koagulation von Blutgefäßen und Lymphabflußbahnen an zwei im Abstand liegenden Stellen durchzuführen und durch den über die ganze Länge des Koagulationsinstrumentes verlaufenden Kanal eine Schneidzange hindurchzuführen und die Gefäße zwischen den beiden Koagulationsstellen zu durchtrennen. Ferner ist ein häufiger, den Arzt und den Patienten belastender Instrumentenaustausch vermieden. Um dabei beim Koagulieren ein Anhaften von Gewebeteilen an den Maulteilen des Koagulationsinstrumente zu vermeiden, ist wenigstens eines der Maulteile mit Ausnehmungen für zuzuführende Spülflüssigkeit versehen, die ein Anhaften von Gewebe oder koagulierten Teilen an den Maulteilen verhindert.

Die Erfindung ist mit weiteren vorteilhaften Merkmalen nachstehend anhand der anliegenden Zeichnungen näher erläutert. Es zeigen:
- Fig. 1: die Seitenansicht eines Mediastinoskopes,
- Fig. 2: einen Achsschnitt eines bipolaren Koagulationsinstrumentes für das Durchführen durch einen Instrumentenkanal des Mediastinoskopes,
- Fig. 2a: eine Seitenansicht auf den distalen Endabschnitt des Instrumentes nach Fig. 2,
- Fig. 3: eine distale Stirnansicht des Instrumentes nach Fig. 2,
- Fig. 4: eine Aufsicht auf das distale Ende des Instrumentes nach Fig. 2,
- Fig. 5: eine durch den Kanal des Koagulationsinstrumentes hindurchführbare Schneidzange.

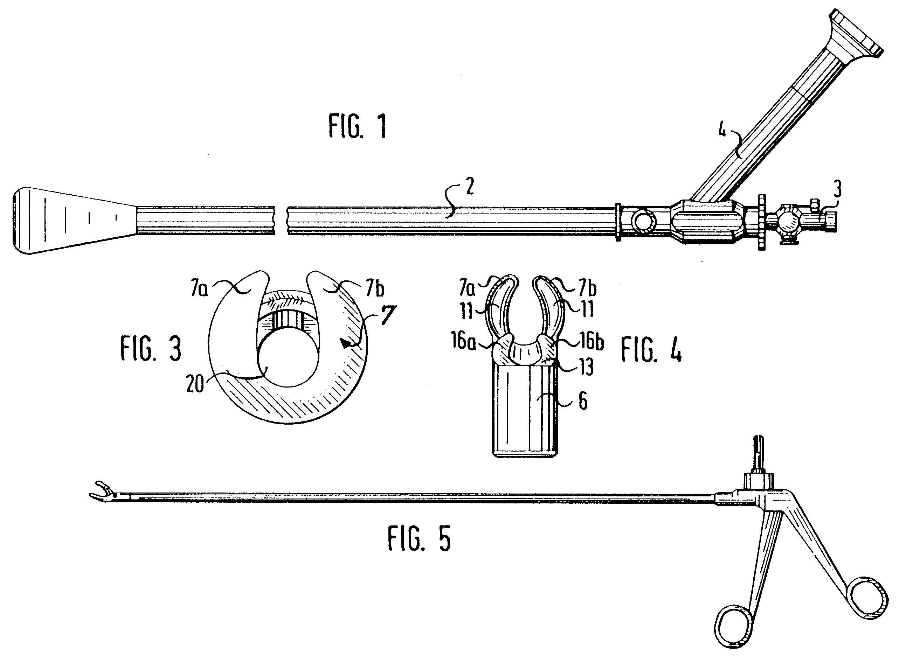

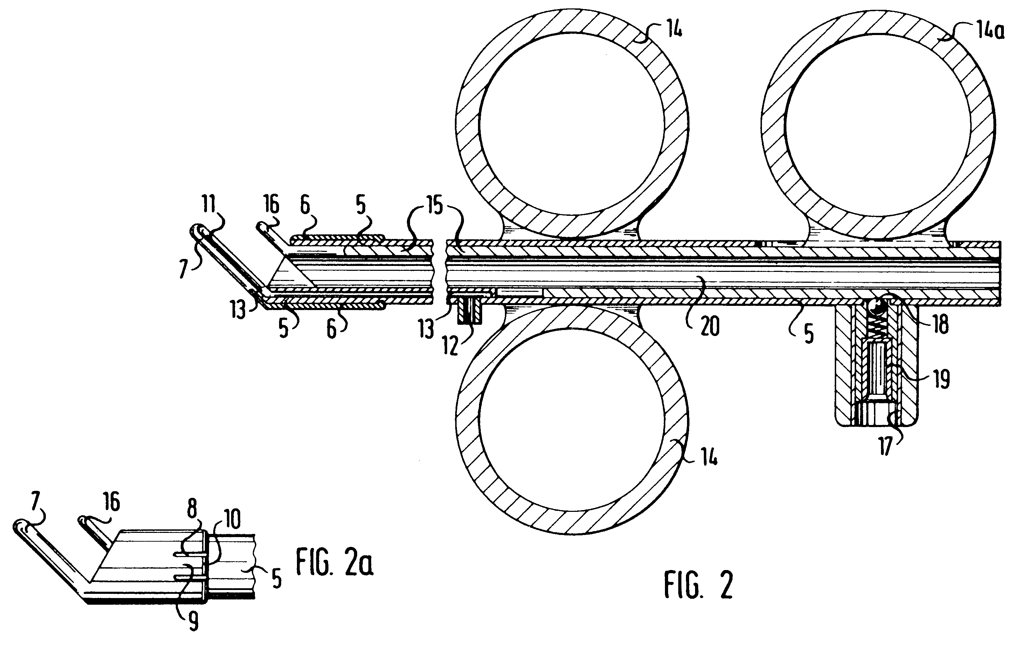

Das bipolare Koagulationsinstrument nach den Fign. 2 bis 4 dient vor allem zum Hindurchführen durch einen Instrumentenkanal 3 eines Mediastinoskopes 2 nach Fig. 1, durch dessen Schaft auch ein Lichtleiterbündel und eine Optik 4 verlaufen. Das durch den Instrumentenkanal des Mediastinoskopes 2 hindurchführende bipolare Koagulationsinstrument nach Fig. 2 und 3 besteht aus einem isolierten Außenschaft 5, auf dessen distalem Ende eine Manschette 6 mit einem ersten, aus zwei im Abstand zueinander angeordneten Hälften 7a, 7b bestehenden Maulteil 7 aufgeschoben und lösbar befestigt ist. Die Befestigung erfolgt z. B. dadurch, daß die Manschette 6 an ihrem proximalen Ende mit achsparallelen Einschnitten 8 versehen ist, durch die federvorgespannte Zungen 9 gebildet werden, die mit einem Wulst an ihren inneren Enden in eine Ringnut 10 des Außenschaftes 5 eingreifen. Die beiden Hälften 7a, 7b des ersten Maulteiles sind mit Ausnehmungen 11 versehen, denen über einen Anschluß 12 und einen Kanal 13 Spülflüssigkeit mittels einer Saug- und Spülpumpe zugeführt wird. In dem mit Griffringen 14 versehenen Außenschaft 5 ist ein Innenschaft 15 durch einen Griffring 14a begrenzt axial verschiebbar vorgesehen. Dieser gegen den Außenschaft 5 isolierte Instrumentenschaft 15 weist am distalen Ende ebenfalls ein abgeschrägtes, zweites Maulteil 16 auf, das aus zwei im Abstand zueinander angeordneten Maulteilhälften 16a, 16b gebildet und parallel zum ersten Maulteil 7 angeordnet ist. Das zweite Maulteil 16 kann ebenfalls Ausnehmungen zum Zuführen einer Flüssigkeit aufweisen (nicht gezeigt).

Der Anschluß der beiden Schäfte 5 und 15 bzw. der Koagulationsmaulteile 7, 16 an eine HF-Stromquelle erfolgt in bekannter Weise dadurch, daß der Außenschaft 5 mit einem Kontakt 17 und der Innenschaft 15 über eine Kontaktkugel 18 oder dergleichen mit einem Kontakt 19 einer Steckverbindung in Verbindung steht.

Wesentlich ist weiter, daß der Innenschaft 15 einen seine gesamte Länge durchlaufenden Instrumentenkanal 20 aufweist. Durch diesen Kanal und durch den Raum zwischen den beiden, jeweils im Abstand zueinander angeordneten Maulteilhälften 7a, 7b, 16a, 16b ist eine an sich bekannte Schneidzange nach Fig. 5 hindurchführbar.

Mit dem bipolaren Koagulationsinstrument wird z. B. in Verbindung mit einem Mediastinoskop folgendermaßen gearbeitet. Zum Freipräparieren der Speiseröhre von umgebendem Gewebe wird zunächst auf der linken Halsseite ein Einschnitt vorgenommen und durch diesen hindurch die Speiseröhre chirurgisch durchtrennt. Ebenfalls erfolgt nach Öffnen des Bauchraumes eine Durchtrennung der Speiseröhre im Bereich des Mageneinganges. Anschliessend wird durch die Speiseröhre von unten, d. h. vom Mageneingang her, eine Sonde hindurchgeführt, die mit dem oberen, umgeschlagenen Ende der Speiseröhre verbunden wird, wonach auf die Sonde ein Zug ausgeübt wird. Dabei erfolgt das Freipräparieren der Speiseröhre durch die durch den Instrumentenkanal 20 hindurchgeführte Schneidzange nach Fig. 5.

Vorhandene Blutgefäße oder Lymphabflußbahnen werden zwischen den beiden, sich jeweils gegenüberliegenden Hälften 7a, 7b, 16a, 16b der beiden Maulteile 7 und 16 festgelegt und durch HF-Stromzufuhr koaguliert. Im Anschluß daran wird der zwischen den beiden Maulteilhälften jedes Maulteiles 7 und 16 liegende Teil des Gefäßes mittels der Schneidzange durchtrennt. Es ist damit möglich, das Koagulationsinstrument nach Fig. 2 bis 4 und die Schneidzange nach Fig. 5 gemeinsam unter visueller Kontrolle durch das Mediastinoskop hindurch zu verwenden, ohne daß ein Austausch von Instrumententeilen zu erfolgen braucht. Es ist auch möglich, die Schneidzange längsverschiebbar im Instrumentenkanal 20 des Koagulationsinstrumente festzulegen, so daß diese eine Funktionseinheit bilden. Das Absaugen von etwaigen Flüssigkeiten in der Körperhöhle kann über den Saug- und Spülkanal im Mediastinoskop erfolgen.

## Patentansprüche

1. Bipolares Koagulationsinstrument zur Verwendung mit einem Mediastinoskop (2) und einer Schneidzange, zum Freipräparieren der Speiseröhre von umgebendem Gewebe, bei dem der Aussenschaft (5) des Koagulationsinstrumentes distal mit zwei zur Längsachse abgewinkelten, im Abstand zueinander angeordneten Maulteilhälften (7a, 7b) eines ersten Maulteiles (7) versehen ist, gegen das die zur Längsachse schräg abgewinkelten, im Abstand zueinander angeordneten Maulteilhälften (16a, 16b) eines zweiten Maulteiles (16) des Innenschaftes (15) des Instrumentes axial verschiebbar sind, und mindestens eines der beiden gegeneinander isolierten Maulteile (7, 16) Ausnehmungen (11) besitzt, die mit einem distal offenen Ende eines Spülkanales (13) verbunden sind, und bei dem durch den Kanal (20) des Innenschaftes und durch den zwischen den beiden Maulteilhälften (7a, 7b) des ersten Maulteiles und den beiden Maulteilhälften (16a, 16b) des zweiten Maulteiles befindlichen Abstand eine Schneidzange oder dergleichen hindurchführbar ist.

2. Instrument nach Anspruch 1, dadurch gekennzeichnet, daß das aus den beiden Hälften (7a, 7b) bestehende erste Maulteil (7) des Außenschaftes (5) mittels einer Manschette (6) auf das distale Ende des Außenschaftes aufschiebbar und daran lösbar festlegbar ist.

3. Instrument nach Anspruch 2, dadurch gekennzeichnet, daß die Manschette (6) an ihrem proximalen Ende mit durch Längsschlitze (8) gebildeten Zungen (9) versehen ist, deren Enden mit einem inneren Ringwulst versehen sind und in eine komplementäre Ringnut (10) des Außenschaftes (5) lösbar eingreifen.

## Claims

1. A bipolar coagulation instrument for use with a mediastinoscope (2) and with a cutting forceps for the free preparation of the oesophagus from the surrounding tissue, in which the outer shaft (5) of the coagulation instrument is provided distally with two jaw piece halves (7a,7b) of a first jaw piece (7), which halves (7a,7b) are angled to the longitudinal axis and are arranged at a distance from each other, towards which the jaw piece halves (16a, 16b), angled obliquely to the longitudinal axis and arranged at a distance from each other, of a second jaw piece (16) of the inner shaft (15) of the instrument are axially displaceable, and at least one of the two jaw pieces (7, 16) which are isolated with respect to each other, has recesses (11) which are connected with a distally open end of a flushing canal (13), and in which a cutting forceps or the like is able to be passed through the canal (20) of the inner shaft and through the space situated between the two jaw piece halves (7a,7b) of the first jaw piece and the two jaw piece halves (16a,16b) of the second jaw piece.

2. An instrument according to Claim 1, characterised in that the first jaw piece (7), consisting of the two halves (7a,7b) , of the outer shaft (5) is able to be pushed by means of a sleeve (6) onto the distal end of the outer shaft and is able to be secured thereon in a releasable manner.

3. An instrument according to Claim 2, characterised in that the sleeve (6) is provided at its proximal end with tongues (9) formed by longitudinal slits (8), the ends of which tongues (9) are provided with an inner annular bead and engage releasably into a complementary annular groove (10) of the outer shaft (5).

## Revendications

1. Instrument de coagulation bipolaire destiné à être utilisé avec un endoscope de médiastinoscopie (2) et une pince de coupe, en vue de dégager l'oesophage en le libérant des tissus environnants, dans lequel le corps-tige extérieur (5) de l'instrument de coagulation est pourvu, côté distal, de deux moitiés de pièce de mâchoire (7a, 7b), recourbées en direction de l'axe longitudinal et disposées à distance l'une de l'autre, d'une première pièce de mâchoire (7), par rapport à laquelle peuvent coulisser axialement les moitiés de pièce de mâchoire (16a, 16b) d'une seconde pièce de mâchoire (16) du corps-tige intérieur (15) de l'instrument, recourbées obliquement en direction de l'axe longitudinal et disposées à distance l'une de l'autre, au moins l'une des deux pièces de mâchoire (7, 16), isolées l'une par rapport à l'autre, possédant des évidements (11) qui sont reliés à une extrémité distale ouverte d'un canal de rinçage (13), et une pince de coupe ou analogue pouvant être passée à travers le canal (20) du corps-tige intérieur et à travers l'intervalle se trouvant entre les deux moitiés de pièce de mâchoire (7a, 7b) de la première pièce de mâchoire et les deux moitiés de pièce de mâchoire (16a, 16b) de la seconde pièce de mâchoire.

2. Instrument selon la revendication 1, caractérisé en ce que la première pièce de mâchoire (7), constituée des deux moitiés (7a, 7b), du corps-tige extérieur (5) peut être emmanchée, au moyen d'un manchon (6), sur l'extrémité distale du corps-tige extérieur, et peut y être fixée de manière amovible.

3. Instrument selon la revendication 2, caractérisé en ce que le manchon (6) est pourvu, à son extrémité proximale, de languettes (9) formées par des fentes longitudinales (8), et dont les extrémités sont munies d'un bourrelet annulaire intérieur et s'engagent de manière amovible dans une rainure annulaire complémentaire (10) du corps-tige extérieur (5).
